# EUROPEAN PATENT APPLICATION

(11) **EP 4 481 028 A1**
(43) Date of publication of application: **25.12.2024**
(21) Application number: 23181173.8
(22) Date of filing: 23.06.2023
(51) Int. Cl.: C12M 1/00, C12M 3/00

(54) **BIOREACTOR FOR TISSUE CULTURE AFTER ADDITIVE MANUFACTURING**

(71) Applicant: Technická Univerzita V Kosiciach, 04200 Kosice-Sever (SK)
(72) Inventor: HUDAK, Radovan, Kosice (SK); FERENCIK, Norbert, Kysta (SK); ZIVCAK, Jozef, Presov (SK); SCHNITZER, Marek, Rozhanovce (SK)

(57) **Abstract**

The bioreactor consists of a chamber with a cell structure installed or an existing used flask, a peristaltic flow pump, a heating body with non-contact heating of the medium, a sensor part with pH measurement (DFROBOT SEN0161), temperature (NTC) and CO2 injection (electromagnetic solenoid). The bioreactor is controlled by a microcomputer (Arduino MEGA), which includes initial visualization and storage of cultivation data. The process is autonomous and protected against possible power outages. It is possible to adapt the conditions based on the requirements with the setting of the required parameters of the pH solution, temperature, or changing the medium in the chamber.

## Description

### Field of invention

The invention of the complete integrated bioreactor system from 3D printed components and the control of the bioreactor system.

### Background to the invention

A tissue engineering bioreactor can be defined as a device that uses mechanical means to influence biological processes. Bioreactors are used in the development of new tissue in vitro by providing biochemical and physical regulatory signals to cells and inducing them to differentiate and/or produce a tissue foundation through the extracellular matrix (intercellular mass) prior to implantation in vivo. Bioreactors can provide biochemical and physical regulatory signals that direct differentiation. There is great potential for the use of mesenchymal stem cells and other multipotent cells to generate cells of a different tissue type. In bioreactors, these processes take place in a strictly monitored and controlled environment. They are sterile containers with temperature regulators and equipment necessary to start the biochemical reaction. The cultivation process requires control of temperature, humidity, pH level, and oxygen level to create the most suitable conditions necessary for maximized cell growth and productivity. In tissue engineering, bioreactors can be used in several areas. In the beginning, we need bioreactors for cell expansion. The cells are intended for direct transplantation. Second, we grow 3D tissues before implantation, such as skin, cartilage, bone, blood vessels, and others, using bioreactors and continuous circulation of the culture medium.

The main types of reactors are:
Nowadays, compression bioreactors have started to be used more widely in the formation of bone and cartilage tissue. By applying dynamic compression, we can simulate the natural environment that naturally acts during the formation of bone or cartilage. Compression bioreactors can improve shape forming and increase the natural elastic modulus when forming cultured tissue.

Bioreactors using hydrostatic pressure are also used for the cultivation of bone and cartilage tissue. These bioreactors consist of culture chambers fully filled with liquid. The cultivation chambers are pressurized using a connected vacuum pump. The vacuum pump injects the culture medium into the bioreactor chamber with an optimal frequency and amplitude. This way, we ensure uniform nutrition of the cultured structure in cooperation with hydrostatic pressure. Hydrostatic pressure ensures more optimal shaping of the bone and cartilage cellular structure, not only on the surface but also inside the cellular carrier. Bioreactors using perfusion have proven extremely successful in forming bone or cartilage tissue. Tissues cultured in a perfusion bioreactor tend to have better viability and product homogenization with biomechanical properties similar to the original tissue. The flow of the cellular suspension is operated using a vacuum pump, but at the same time, it is regulated by the used flow meter. With the help of perfusion, the deposition of chondrocytes or osteocytes in a cellular carrier is significantly more efficient.

Tensile bioreactors were developed mainly for the production of tendon and ligament tissue. Some devices are very similar to compressor bioreactors, with the difference that instead of compression force, tensile force is used. Collagen-glucosaminoglycan scaffolds with injected cells are anchored using clamps to an environment in which uniformly changing, one-dimensional, tensile forces act. Due to the influence of these forces, mesenchymal stem cells show differentiation in the optimal direction and with optimal properties for the formation of connective tissue.

The last group is rotating bioreactors, which were first developed at NASA to protect cell cultures from the heavy loads that can damage them during takeoff or landing of a spacecraft. In the rotating bioreactor, the scaffolds move freely without any fixation in the culture vessel. The vessel is cylindrical in shape and rotates about its longitudinal axis at a constant angular velocity. As the vessel rotates, the vessel wall exerts an upward hydrodynamic buoyancy force that balances the downward gravitational force, so that the scaffold remains virtually floating in the fluid while there is a constant laminar flow at the edges. This dynamic laminar flow effectively stimulates the transport of nutrients and oxygen.

### References:

ROTONDI, Marco, et al. Design and development of a new ambr250® bioreactor vessel for improved cell and gene therapy applications. Biotechnology Letters, 2021, 43.5: 1103-1116.
RADJENOVIC, Jelena; PETROVIC, Mira; BARCELONA, Damiá. Analysis of pharmaceuticals in wastewater and removal using a membrane bioreactor. Analytical and bioanalytical chemistry, 2007,
   387.4: 1365-1377.
CORPUZ, Mary Vermi Aizza, et al. Wastewater treatment and fouling control in an electro algae-activated sludge membrane bioreactor. Science of the Total Environment, 2021, 147475.
ZABLOTSKII, Vitalii; POLYAKOVA, Tatyana; DEJNEKA, Alexander. Cells in the non-uniform magnetic world: how cells respond to high-gradient magnetic fields. BioEssays, 2018, 40.8: 1800017.
ZABLOTSKII, Vitalii; POLYAKOVA, Tatyana; DEJNEKA, Alexander. Modulation of the Cell Membrane Potential and Intracellular Protein Transport by High Magnetic Fields. Bioelectromagnetics,
   2021, 42.1: 27-36.
GURHAN, Hakki, et al. Effects induced by a weak static magnetic field of different intensities on HT-1080 fibrosarcoma cells. Bioelectromagnetics, 2021, 42.3: 212-223.
NAKHAEI, Kourosh, et al. Ultraviolet Light Treatment of Titanium Enhances Attachment, Adhesion,
   and Retention of Human Oral Epithelial Cells via Decarbonization. Materials, 2021, 14.1: 151.
SKOPELJA-GARDNER, Sladjana, et al. Acute skin exposure to ultraviolet light triggers neutrophil-
   mediated kidney inflammation. Proceedings of the National Academy of Sciences, 2021, 118.3.
SORO, Arturo B., et al. Modeling the effect of UV light at different wavelengths and treatment combinations on the inactivation of Campylobacter jejuni. Innovative Food Science & Emerging Technologies, 2021, 69: 102626.
BORRA RC, Lotufo MA, Gagioti SM, Barros Fde M, Andrade PM. A simple method to measure cell viability in proliferation and cytotoxicity assays. Braz Oral Res. 2009 Jul-Sep;23(3):255-62. doi: 10.1590/s1806-83242009000300006. PMID: 19893959.
BRANDÃO, Hugo B.; GABRIELE, Michele; HANSEN, Anders S. Tracking and interpreting long-range chromatin interactions with super-resolution live-cell imaging. Current Opinion in Cell Biology,
   2021, 70: 18-26.

### Summary of the invention

We currently have a flow bioreactor with a flow chamber and complex control. The bioreactor consists of a chamber with a cell structure installed or an existing used flask, a peristaltic flow pump, a heating body with non-contact heating of the medium, a sensor part with pH measurement (DFROBOT SEN0161), temperature (NTC) and CO2 injection (electromagnetic solenoid). The bioreactor is controlled by a microcomputer (Arduino MEGA), which includes initial visualization and storage of cultivation data. The process is autonomous and protected against possible power outages. It is possible to adapt the conditions based on the requirements with the setting of the required parameters of the pH solution, temperature, or changing the medium in the chamber.

In order to establish the correct conditions in the development chamber of the bioreactor, the following parameters must be monitored, which are also controlled in our system using feedback control:
- the temperature of the nutrient medium, controlled by non-contact heating,
- the flow of the medium through the bioreactor chamber, controlled by a peristaltic pump,
- subsystem for monitoring and controlling pH and CO2 content in the nutrient solution,
- changing the position of the cells in relation to gravity, carried out by tilting the entire bioreactor chamber,
- delivery of the required substances and antibiotics into the solution of the nutrient medium without contaminating the solution,
- the possibility of inserting the bioreactor chamber into radiological observation devices, ensured by the construction of the chamber,
- the possibility of monitoring cell development in real-time, provided by a microscopic camera for monitoring processes inside the bioreactor,
- parameter change during cultivation and control, controlled by a microcomputer with visualization on a tablet with a touch function,
- exchange of the medium after a set time based on the properties and specificity of the cells, controlled by a secondary peristaltic pump and microcomputer.

During cultivation, an acute exchange of the nutrient medium in the chamber is possible without interrupting the cultivation process. It is also possible to enrich the medium with an antibiotic component and other nutrients. During cultivation, the sample can be observed with a digital microscopic camera with a magnification of 2000x. Good cell proliferation is ensured by the tilt of the chamber, which is also adjustable. The entire bioreactor system is placed in a sterile box.

In the current course of testing the mentioned integrated bioreactor system, we used gingival fibroblasts, which form the soft connective tissue that surrounds the alveolar bone (gingiva). Meanwhile, periodontal ligament fibroblasts surround the root portion of the tooth, producing and maintaining a connective tissue implant that provides stable anchorage. During the tests on our solution, we found that with excessive and frequent tilting of the chamber, the adhesion of the cells is lost, and they are gradually released, and since the flow through the chamber is constant, the cells get into the circulation system of the reactor. Tilting the bioreactor is not necessary for all cell types that are cultured. Further chamber testing will consist of two closed circuits, one of which is nutritional and does not come into contact with the cells. A peristaltic pump circulates the medium through the heating block into the medium reservoir, where it is enriched with CO2 and possible antibiotics. Stabilizing elements were already added to the medium during the preparation of the medium. Subsequently, the second peristaltic pump adds a new medium to the flask containing the cells at selected intervals. Such a sequence will prevent cells from washing away from the adhesive surface of the bioreactor flask.

### Brief description of the drawings

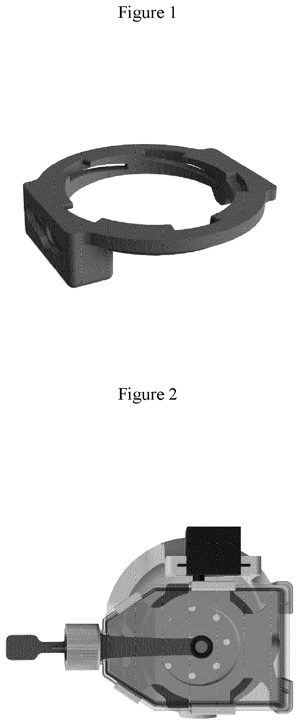

Fig. 1 - Chamber fixator in the tilting cradle of the reactor with attachment to the servo motor (PA12 material)
Fig. 2 - Fixed flask with attached structure (top view (servomotor is shown in black))

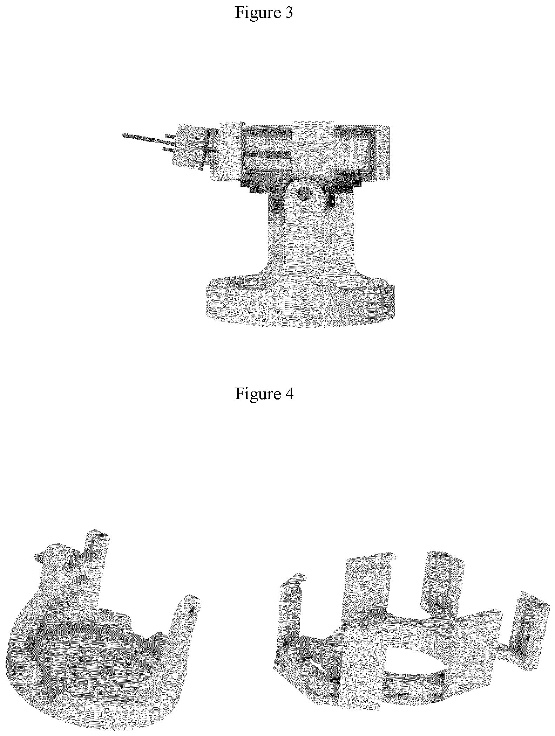

Fig. 3 - Fixed flask with attached structure (side view)
Fig. 4 - Part of the reactor called the cradle (on the left (PA12 material)) parts of the reactor used to fix the flask (on the right (PA12 material))

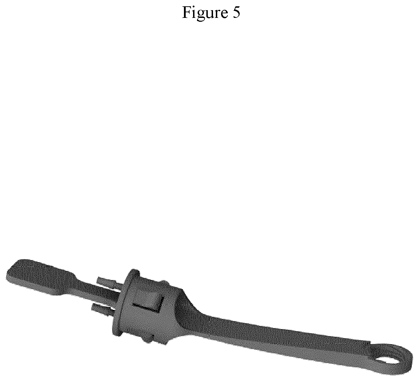

Fig. 5 - A spoon with a hole for the scaffold and holenders for feeding and withdrawing the nutrient medium from the flask (material Resin)

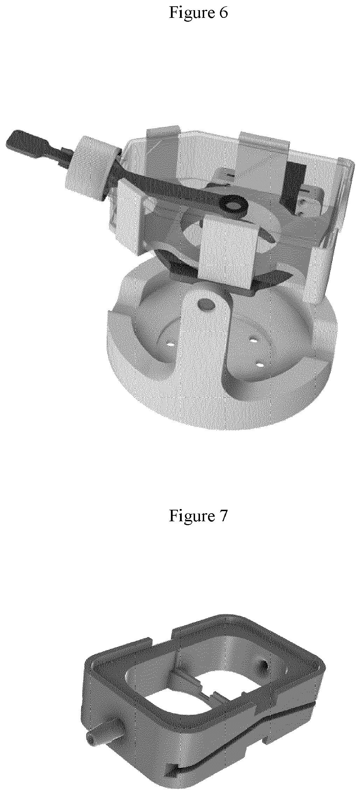

Fig. 6 - Complete reactor (side view)
Fig. 7 - Chamber with bayonet attachment and central cell handle (replaceable with a flask (Resin material))

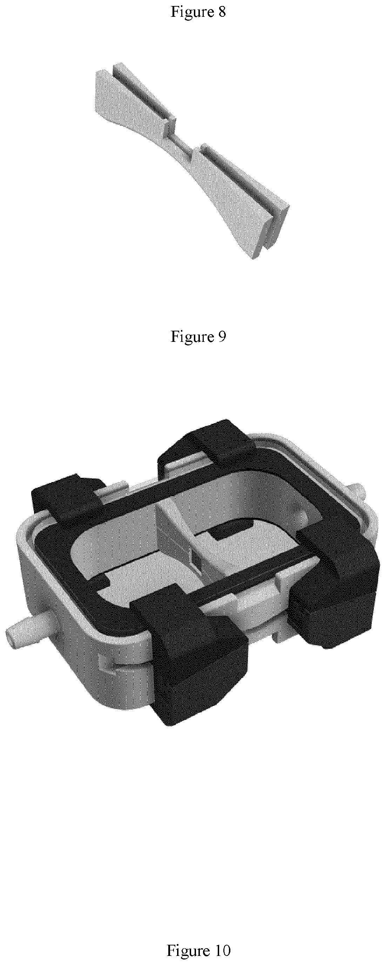

Fig. 8 - Central handle of the cells (intended for the bayonet chamber, not for the flask (material Resin))
Fig. 9 - Complete bayonet chamber (side view (material Resin))

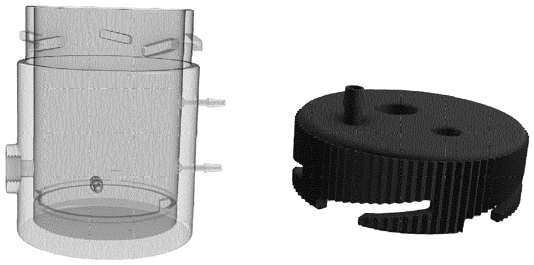

Fig. 10 - Medium reservoir for the bioreactor with holenders for medium supply and outlet (material Resin)

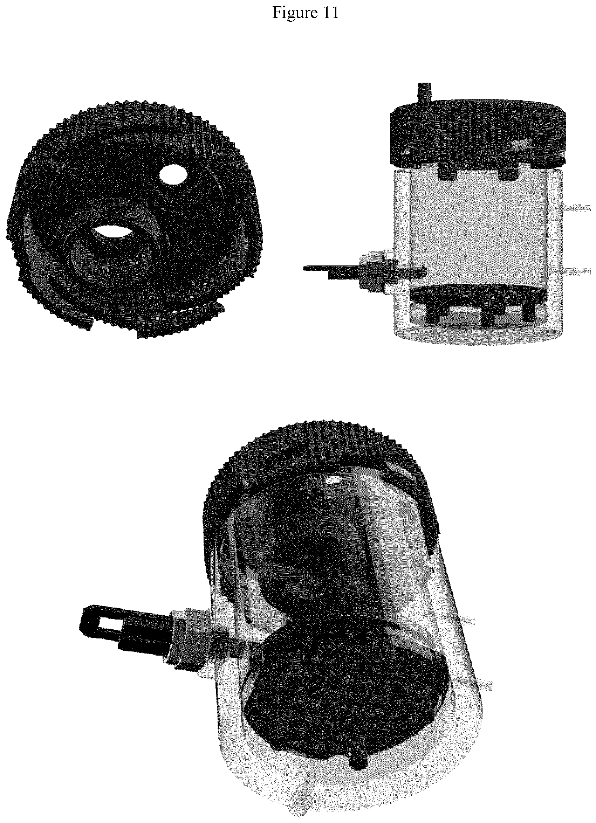

Fig. 11 - Complete medium reservoir chamber with built-in thermometer, CO2 dissolving pad and lid (Resin and PA12 material)
Fig. 12 - Heating block for non-contact medium (material Aluminium and PETG)
Fig. 13 - Built-in peristaltic pump

### Detailed description

The obtained results have a wide range of use in practice in the creation of artificial cell structures, tissue, and organ structures. With the help of the bioreactor platform, we have the possibility of cultivating cells and subsequently creating flexible cell or tissue structures through an automated process that will increase the quality of cell proliferation and viability. The process can also be used to simulate the effect of substances on already existing cell structures or to determine the compatibility of substances with an optional cell structure. The application of the developed system to the pharmacological, and dermatological industry, respectively, is therefore significant as a replacement for part of the clinical or animal tests. With the help of a bioreactor, we can establish special procedures for the formation of cell structures in in vivo conditions, which cannot otherwise be simulated in the environment of a standard CO2 incubator. Furthermore, we consider it as useful possibility to influence cells during their development and proliferation in the bioreactor chamber with simultaneous observation using a microscopic camera. The state of the cells can be influenced by changing the temperature, flow rate, changing the pH of the solution based on the CO2 content, and changing the chamber tilt. In our case, the double circulation is also unique, which takes care of the correct quality of the medium on the media preparation side and can be added to the cells at any interval. In this way, the quality of cell proliferation and the subsequent health of the cells for their further use can be significantly influenced.

## Claims

1. A universal bioreactor system, made from 3D printed components of biocompatible materials **characterized in that** the chamber can be moved in any direction and axis during cultivation.

2. A universal bioreactor system, made from 3D printed components of biocompatible materials according to claim 1 **characterized in that** it is autonomous without interference from the external environment and controlled by an ATmega 2560- based microcomputer.

3. A universal bioreactor system, made from 3D printed components of biocompatible materials according to claim 1 **characterized in that** it is universal for every commonly used flask intended for cultivation.

4. A universal bioreactor system, made from 3D printed components of biocompatible materials according to claim 1 **characterized in that** it contains two cyclic branches in which the medium is selectively added to the flask with cells.

5. A universal bioreactor system, made from 3D printed components of biocompatible materials according to claim 1 **characterized in that** it does not contain metal parts in the cultivation area and can be observed during cultivation using radiological methods.

6. A universal bioreactor system, made from 3D printed components of biocompatible materials according to claim 1 **characterized in that** the cells are mounted on a scaffold fixator made of E-Shell 300 series material resin, which is CE certified and has biocompatibility type class II according to ISO 10993 (standard on medical products).

7. A universal bioreactor system, made of components created from 3D prints of biocompatible materials according to claim 1 **characterized in that** the components for tilting the chamber, heating the medium, and the medium supply are modular and adaptable for different sizes of the flask used.

8. A universal bioreactor system, made from 3D printed components of biocompatible materials according to claim 1 **characterized in that** cells can be observed microscopically even during cultivation, unlike standard cultivation in an incubator.

9. A universal bioreactor system, made from 3D printed components of biocompatible materials according to claim 1 **characterized in that** the exchange of the nutrient medium intended to nourish the cells and to support the development of the structure is automated based on the colour change.

10. A universal bioreactor system, made from 3D printed components of biocompatible materials according to claim 1 **characterized in that** the cultivation can be changed remotely, and its conditions can be observed using the IoT cloud system.

11. A universal bioreactor system, made of 3D printed components of biocompatible materials according to claim 1 **characterized in that** the scaffold on which the cells are adhered is made of biocompatible recyclable material based on PLA, PHB and ceramics.
